# EUROPÄISCHE PATENTSCHRIFT

(11) **EP 1 490 322 B1**
(45) Veröffentlichungstag und Bekanntmachung des Hinweises auf die Patenterteilung: **29.07.2015**
(21) Anmeldenummer: 03708200.5
(22) Anmeldetag: 10.03.2003
(51) Int. Cl.: C07C 67/22, C07C 69/65

(54) **VERFAHREN ZUR HERSTELLUNG VON 2,2-DICHLORO- ODER DIBROMO-PHENYLESSIGSÄUREALKYLESTERN**
METHOD FOR PRODUCING 2,2-DICHLORO OR DIBROMO-PHENYL ALKYL ACETATES
PROCEDE POUR REALISER DES ACETATES D'ALKYLE 2,2-DICHLORO- OU DIBROMO-PHENYLE

(30) Priorität: 04.04.2002 AT 5232002
(43) Veröffentlichungstag der Anmeldung: 29.12.2004
(73) Patentinhaber: DSM Fine Chemicals Austria Nfg GmbH & Co KG, 4021 Linz (AT)
(72) Erfinder: KRICH, Sylvia, A-4203 Altenberger bei Linz (AT); HOLUB, Bernhard, A-4072 Strassham (AT)
(74) Vertreter: Verhaegen, Ilse Maria M.
(86) Internationale Anmeldenummer: PCT/EP2003/002407
(87) Internationale Veröffentlichungsnummer: WO 2003/084918

(56) Entgegenhaltungen:
- EP-A- 0 075 356
- EP-A- 0 518 412
- DE-A- 2 827 977

## Beschreibung

2,2-Dichloro-oder Dibromo-phenylessigsäurealkylester werden als Intermediate beispielsweise bei der Herstellung von Pestiziden oder als Vulkanisationsbeschleuniger für Elastomere eingesetzt.

Die Herstellung dieser Verbindungen erfolgte anfänglich, wie beispielsweise in EP 0 075 356 beschrieben wird, durch Reaktion von Phosphorpentachlorid mit einem Phenylglyoxylsäureester, der aus Benzoylcyanid erhalten wurde. Da Benzoylcyanid ein sehr teures Edukt war, wurde als Alternative Benzylcyanid als Edukt für die Herstellung von 2,2-Dichlorphenylacetonitril verwendet, beispielsweise analog EP 0 518 412, das sodann gemäß EP 0 075 356 in den gewünschten 2,2-Dichlorophenylessigsäurealkylester überführt wird.

Dabei wird 2,2-Dichlorphenylacetonitril mit Wasser und einem Alkohol in Gegenwart eines Halogenwasserstoffes, bevorzugt gasförmige HCl, bei einer Temperatur von 0 bis 80°C, bevorzugt von 15 bis 50°C umgesetzt.

Ein Problem bei der in EP 0 075 356 beschriebenen Verfahrensweise ist die Bildung des Nebenproduktes 2,2-Dichlorphenylacetamid, das sodann aus dem Reaktionsgemisch abgetrennt werden muss und die Ausbeute an der gewünschten Endverbindung deutlich mindert. Ein weiteres Problem ist, bei Verwendung von Ethanol als Alkohol und HCl, die Bildung von Ethylchlorid, einem giftigen, leicht entzündlichem Nebenprodukt, das nicht emittiert werden darf. Weitere Nebenreaktionen sind die Hydrolyse des Endproduktes zum entsprechenden Phenylglyoxylsäureester bzw. zu Phenylglyoxylsäure. Um den Spezifikationen für die Weiterverarbeitung zu genügen, dürfen diese Nebenprodukte, wie etwa Phenylglyoxylsäure, jedoch nur in äußerst geringen Mengen im Endprodukt vorhanden sein.

Bei der Herstellung der 2,2-Dichlorophenylessigsäurealkylester unter den in EP 0 075 356 beschriebenen Parametern und Verfahrensablauf wird das gewünschte Endprodukt zudem in Ausbeuten von lediglich bis zu 75% und mit hohen Anteilen an den verschiedensten Nebenprodukte erhalten.

Aufgabe der vorliegenden Erfindung war es demnach ein verbessertes Verfahren zur Herstellung von 2,2-Dichloro- oder Dibromo-phenylessigsäurealkylestern zu finden, das die gewünschten Produkte in höheren Ausbeuten und mit höherer Reinheit erhältlich macht.

Gegenstand der Erfindung ist demnach ein verbessertes Verfahren zur Herstellung von 2,2-Dichloro- oder Dibromo-phenylessigsäurealkylester der Formel 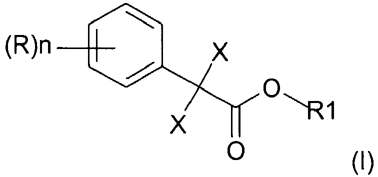 in der X Cl oder Br ist, n eine ganze Zahl von 1 bis 5 sein kann, R Wasserstoff, C₁-C₈-Alkyl, Aryl, Heteroaryl, C₁-C₈-Alkoxy, Aryloxy oder Halogen und R1 C₁-C₈-Alkyl, bedeuten, das dadurch gekennzeichnet ist, dass ein 2,2-Dichlor- oder Dibromphenylacetonitril der Formel 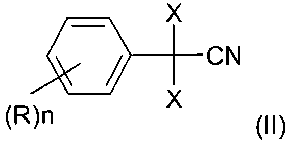 in der X, n und R wie oben definiert sind,

in 0,8 bis 2 mol Wasser pro mol Nitril der Formel (II), 1 bis 8 mol Alkohol der Formel

R1OH (III)

in der R1 wie oben definiert ist, pro mol Nitril der Formel (II) und

in Gegenwart von 1 bis 3 mol HCl oder HBr pro mol Nitril der Formel (II), gegebenenfalls in Anwesenheit eines unter den Reaktionsbedingungen inerten Lösungsmittels zu dem entsprechenden 2,2-Dichloro- oder Dibromo-phenylessigsäurealkylester der Formel (I) umgesetzt wird, wobei die Reaktionstemperatur in der ersten Phase der Umsetzung bei 35 bis 55°C und in der zweiten Phase bei 60 bis 100°C liegt worauf nach erfolgter Umsetzung das Reaktionsgemisch auf 20 bis 40°C abgekühlt, mit Wasser verdünnt wird, und der entsprechende 2,2-Dichloro- oder Dibromo-phenylessigsäurealkylester der Formel (I) isoliert wird.

Bei dem erfindungsgemäßen Verfahren wird ein 2,2-Dichlor- oder Dibromphenylacetonitril der Formel (II) mit Wasser, Alkohol R1OH und HCl oder HBr, gegebenenfalls in Anwesenheit eines unter den Reaktionsbedingungen inerten Lösungsmittels zu dem entsprechenden 2,2-Dichloro- oder Dibromo-phenylessigsäurealkylester der Formel (I) umgesetzt,

In der Formel (II) bedeutet X Chlor oder Brom, bevorzugt Chlor,
n steht für eine ganze Zahl von 1 bis 5.

R kann dabei Wasserstoff, C₁-C₈-Alkyl, Aryl, Heteroaryl, C₁-C₈-Alkoxy, Aryloxy oder Halogen bedeuten.

Unter C₁-C₈-Alkyl sind dabei lineare oder verzweigte Alkylreste mit 1 bis 8 C~Atomen, wie etwa Methyl, Ethyl, i-Propyl, n-Propyl, n-Butyl, tert,-Butyl, n-Propyl, Hexyl oder Octyl, zu verstehen.

Bevorzugt sind lineare oder verzweigte C₁-C₄-Alkylreste.

Unter C₁-C₈-Alkoxy sind Alkoxyreste mit 1 bis 8 C-Atomen zu verstehen, wobei der Alkylteil linear oder verzweigt sein kann, wie etwa Methyloxy, Ethyloxy, i-Propyloxy-, n-Propyloxy, n-Butyloxy, tert.-Butyloxy, n-Propyloxy, Hexyloxy oder Octyloxy. Bevorzugt sind lineare oder verzweigte C₁-C₄-Alkoxyreste.

Unter Aryl und Aryloxy sind bevorzugt aromatische Reste mit 6 bis 20 C-Atomen, wie Phenyl, Biphenyl, Naphthyl, Indenyl, Fluorenyl, Phenoxy u.s.w. zu verstehen. Bevorzugt sind als aromatische Reste Phenyl und Phenoxy.

Unter Heteroaryl sind aromatische Reste, die zumindest ein S-, O- oder N-Atom im Ring oder Ringsystem enthalten. Dies sind beispielsweise Furyl, Pyridyl, Pyrimidyl, Thienyl, Isothiazolyl. Imidazolyl, Chinolyl, Benzothienyl, Indolyl, Pyrrolyl u.s.w..

Unter Halogen sind Fluor, Chlor, Brom und Iod zu verstehen, wobei Fluor, Brom und Chlor bevorzugt sind.

Falls R nicht Wasserstoff ist, darin bedeutet n bevorzugt eine ganze Zahl von 1 bis 3, besonders bevorzugt 1 oder 2.

Bevorzugt bedeutet R Wasserstoff, einen unsubstituierten, linearen oder verzweigten C₁-C₄-Alkyl- oder Alkoxyrest, unsubstituiertes Phenyl oder Phenoxy, oder Chlor. Besonders bevorzugt bedeutet R Wasserstoff.

Die für das erfindungsgemäße Verfahren eingesetzten Edukte der Formel (II) sind käuflich erwerbbar oder können beispielsweise aus Benzylcyanid, etwa gemäß EP 0 518 412, hergestellt werden.

Die Nitrile der Formel (II) werden erfindungsgemäß mit 0,8 bis 2 mol Wasser pro mol Nitril der Formel (II), 1 bis 8 mol Alkohol der Formel R1OH (III) pro mol Nitril der Formel (II) und 1 bis 3 mol HCl bzw. HBr pro mol Nitril der Formel (II) umgesetzt.

Als Alkohol der Formel (III) eignen sich solche worin R1 C₁-C₈-Alkyl bedeutet. Beispiele dafür sind Methanol, Ethanol, n-Propanol, i-Propanol, i-Butanol, n-Pentanol oder n-Hexanol.

Die Auswahl des Alkohols hängt dabei vom gewünschten Ester im Endprodukt ab. Bevorzugt wird als Alkohol der Formel (III) Methanol, Ethanol oder n-Butanol, besonders bevorzugt Ethanol eingesetzt.

Der Alkohol der Formel (III) wird dabei in einer Menge von 1 bis 8 mol pro mol Nitril der Formel (II), bevorzugt von 3 bis 5 mol pro mol Nitril der Formel (II), eingesetzt. Größere Mengen an Alkohol können gewünschtenfalls auch eingesetzt werden, sind jedoch aus wirtschaftlichen Gründen nicht sinnvoll.

Wird der Alkohol in einer Menge von 1 bis etwa 3 mol pro mol Nitril eingesetzt, so ist es von Vorteil ein zusätzliches, unter den Reaktionsbedingungen inertes Lösungsmittel einzusetzen. Geeignete Lösungsmittel sind beispielsweise Ether, wie etwa Methyl-tert.butylether (MTBE), Diethylether, Tetrahydrofuran (THF), Dioxan oder höhere Ether, wie etwa Ethylenglykoldimethylether u.s.w. oder gegebenenfalls halogenierte Kohlenwasserstoffe, wie etwa Toluol, Hexan, Heptan, Dichlormethan, Chlorbenzol u.s.w..

Wasser wird dem Reaktionsgemisch in einer Menge von 0,8 bis 2 mol pro mol Nitril der Formel (II), bevorzugt von 0,9 bis 1,5 mol pro mol Nitril der Formel (II), zugesetzt.

Weiters werden 1 bis 3 mol HCl bzw. HBr pro mol Nitril der Formel (II) zugegeben.

Bei dem erfindungsgemäßen Verfahren kann das Nitril der Formel (II) zuerst in dem gewünschten Alkohol der Formel (III), gegebenenfalls einem unter den Reaktionsbedingungen inerten Lösungsmittel und in Wasser gelöst werden, worauf anschließend gasförmige HCl bzw. HBr eingeleitet wird. Als gasförmige HCl bzw. HBr kann dabei auch das bei der Chlorierung mit Chlorgas, bzw. Bromierung mit Br₂ von gegebenenfalls substituierten Benzylcyanid zu den gewünschten Nitrilen der Formel (II), beispielsweise gemäß EP 0 518 412, erhaltenen HCl- bzw. HBr-Abgas eingeleitet werden, wodurch eine direkte Kopplung der Herstellung des Nitrileduktes der Formel (II) an die Herstellung des gewünschten Dichloro- oder Dibromo-phenylessigsäurealkylesters der Formel (I) erzielt werden kann.

Ein weiterer Gegenstand der Erfindung ist demnach ein Verfahren zur Herstellung von 2,2-Dichloro- oder Dibromo-phenylessigsäurealkylester der Formel (I), das dadurch gekennzeichnet ist, dass in einer 1. Stufe ein gegebenenfalls substituiertes Benzylcyanid der Formel 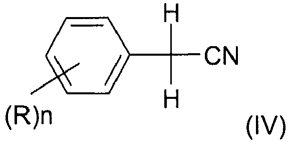 mit Chlor, in Gegenwart katalytischer Mengen an Salzsäuregas, bzw. mit einem Bromierungsmittel zu dem korrespondierenden Nitril der Formel (II) umgesetzt wird, und das sich bildende HCl- bzw. HBr-Abgas in der zweiten Stufe zur Umsetzung des Nitrils der Formel (II) zu dem entsprechenden 2,2-Dichloro- oder Dibromophenylessig-säurealkylester der Formel (I) eingesetzt wird.

Stufe 1 kann dabei analog EP 0 518 412 durchgeführt werden.

In einer bevorzugen Variante wird für die Umsetzung des Nitrils der Formel (II) ein Alkohol/Wasser/HCl-bzw. HBr-Gemisch verwendet.

Dieses Dreiergemisch kann dadurch erhalten werden, dass in eine Mischung aus Wasser und Alkohol Salzsäuregas bzw. HBr eingeleitet wird.

Besonders bevorzugt wird das Alkohol/Wasser/HCl-bzw, HBr-Gemisch dadurch erhalten, dass das bei der Halogenierung mit Cl₂ oder Br₂ von gegebenenfalls substituierten Benzylcyaniden zu den gewünschten Nitrilen der Formel (II), beispielsweise gemäß EP 0 518 412, erhaltene HCl-bzw. HBr-Abgas in ein Gemisch aus Wasser und Alkohol geleitet wird. Dies hat den Vorteil, dass die Herstellung des Nitrils der Formel (II) nicht direkt mit der Herstellung des Dichloro-bzw. Dibromo-phenylessigsäurealkylesters der Formel (I) gekoppelt werden muss. HCl bzw. HBr, die als Abgas aus der Halogenierung erhalten werden, können somit auch in Form einer wässrig, alkoholischen Lösung zwischengelagert werden.

Das Gemisch kann aber auch durch Einleiten von HCl oder HBr, bzw. von HCl oder HBr-Abgas in eine Lösung aus Alkohol und wässriger HCl bzw. HBr oder in Alkohol mit anschließendem Verdünnen mit Wasser erhalten werden.

Das gewünschte Molverhältnis im Drelergemisch kann gegebenenfalls durch Verdünnen der vorhandenen wässrig, alkoholischen HCl- bzw. HBr-Lösung mit. Alkohol und/oder Wasser eingestellt werden.

Wird bei dem erfindungsgemäßen Verfahren ein Alkohol/Wasser/HCl-bzw. HBrGemisch eingesetzt, so kann zuerst das Nitril der Formel (II) vorgelegt und anschileßend das Dreiergemisch zudosiert werden. Es kann jedoch auch das Dreiergemisch vorgelegt und dann das Nitril der Formel (II) zugegeben werden.

Bei dem erfindungsgemäßen Verfahren erfolgt die Umsetzung in der ersten Phase bei einer Temperatur von 35 bis 55°C.

Dazu wird beispielsweise entweder das Nitril der Formel (II) oder das Dreiergemisch vorgelegt, auf 35 bis 55°C erwärmt und anschließend bei dieser Temperatur die fehlende Reaktionskomponente zudosiert. Das so erhaltenen. Reaktionsgemisch wird sodann noch für einige Minuten bis zu mehreren Stunden, bevorzugt von 30 Minuten bis zu 5 Stunden, bei dieser Temperatur gerührt.

In der zweiten Phase wird das Reaktionsgemisch auf 60 bis 100°C, bevorzugt auf 65 bis 80°C erhitzt und wiederum noch für einige Minuten bis zu mehreren Stunden, bevorzugt von 30 Minuten bis zu 10 Stunden, bei dieser Temperatur gerührt.

Nach Kontrolle der vollständigen Umsetzung wird das Reaktionsgemisch auf 20 bis 40°C abgekühlt und soviel Wasser zugesetzt, dass das ausgefallene Ammoniumchlorid oder -bromid gerade gelöst wird und eine Phasentrennung eintritt. Anschließend kann die wässrigen Phase gewünschtenfalls mittels üblicher Extraktionsmittel, wie etwa Hexan, Heptan, Toluol, Ether, oder Estern extrahiert werden. Der Extrakt wird dann mit der organischen Phase vereinigt.

Die Isolierung des Endproduktes kann beispielsweise dadurch erfolgen, dass zunächst bei Normaldruck und einer Temperatur bis max, 90°C ggf. Extraktionsmittel, Wasser und Alkohol und sodann unter Vakuum Leichtsieder bzw. die Nebenprodukte Phenylglyoxylsäure, Phenylglyoxylsäureethylester, Phenylessigsäureethylester (aus unvollständiger Halogenierung) und Monochlor- oder Monobromphenylessigsäureester, bis zu einem Erreichen einer konstanten Siedetemperatur abdestilliert werden, sodass das entsprechende Endprodukt der Formel (I) im Sumpf verbleibt. Zur weiteren Reinigung kann das Produkt über Kopf destilliert werden. Nach Vereinigung der organischen Phasen kann jedoch auch zuerst das Wasser am Wasserscheider abgetrennt werden und dann bei Normaldruck Alkohol und ggf. Extraktionsmittel abdestilliert werden.

Einhält das rohe Endprodukt noch zuviel organische Säuren, die durch die Hydrolyse des Produktes entstehen, so wird das Rohprodukt noch einmal mit einem der oben ungeführten Extraktionsmittel und Alkohol versetzt und erneut destillativ aufgearbeitet, wobei eine Rückveresterung erfolgt.

Durch das erfindungsgemäße Verfahren werden 2,2-Dichloro- oder Dibromophenylessig-säurealkylester der Formel (I) im Vergleich zum Stand der Technik höheren Ausbeuten und höheren Reinheiten erhalten, wobei wesentlich weniger Emissionen als bei bisher üblichen Verfahren entstehen und zudem durch die Abgasverwertung weniger Rohstoffe benötigt werden.

### Beispiel 1: Laborversuch Chlorierung Benzylcyanid zu 2,2-Dichlorphenylacetonitril

1436g (12,27 mol) Benzylcyanid wurden in einen zuvor inertisierten Email-Autoklaven chargiert. Danach wurden 239g (6,55 mol = 0,533 Äq.) Salzsäuregas bei geöffnetem Abgasventil eingeleitet, danach das Abgasventil geschlossen und auf 40°C erwärmt.

Anschließend wurden 1830g (25,77 mol = 2,10 Äq.) Chlor über 6 Stunden bei 60-65°C und 3 bar eingeleitet. Dabei stieg die Temperatur innerhalb der ersten Minuten rasch an, blieb dann aber in Abhängigkeit von der Einleitgeschwindigkeit annähernd konstant. Bei Erreichen eines Innendruckes von 3 bar wurde das Abgasventil leicht geöffnet, sodass der Druck konstant bei 3 bar blieb.

Gegen Ende der Reaktion sanken Temperatur und Druck leicht ab. Nach Ende des Chloreinleitens wurde das Abgasventil geschlossen und 30 Minuten bei 55°C gerührt.

Danach wurde der Autoklav entspannt, und zum Austreiben von Chlor und HCl-Gas Stickstoff durchgeleitet.

Der Abgaswäscher wurde mit 2823g (61,3 mol = 5Äq.) Ethanol und 340g konzentrierter Salzsäure (12,27 mol = 1 Äq. entsprechend 221g Wasser und 3,26 mol = 0,266 Äq. = 119g Salzsäure) beschickt und das Abgas aus der Chlorierung bei 10-15°C zu 95% (solange kein Chlor (max. 3%) im Abgas war) eingeleitet. Dahinter war ein Sicherheitswäscher (mit 10%iger Natronlauge beschickt) geschaltet.

Das restliche Abgas wurde in 2 hintereinander geschaltene Wäscher mit 10%iger Natronlauge eingeleitet.

### Ausbeute:

2200g 2,2-Dichlorphenylacetonitril (96,3% d.Th.)
3950g 24,5%ige ethanolische Salzsäure (Ausbeute an HCl aus der Chlorierung: 73% d.Th.)

### Beispiel 2: Herstellung von 2,2-Dichlorphenylessigsäureethylester aus 2,2-Dichlorphenylacetonitril (2. Stufe Labor (Variante I))

70,0g (0,38 Mol) destilliertes 2,2-Dichlorphenylacetonitril, hergestellt nach Beispiel 1, wurden auf 40°C erwärmt.

Dann wurden innerhalb von 30 Minuten 123,57g einer aus einer Chlorierung analog Beispiel 1 erhaltenen ethanolischen Salzsäure (30,22g = 0,83 mol entsprechend 2,18Äq. Salzsäure, 6,77g = 3,76 mol = 0,99 Äq. Wasser und 86,6g = 1,88 mol = 4,95 Äq. Ethanol) bei 40°C zudosiert und nach erfolgter Zugabe weitere 2 Stunden bei 40°C gerührt. Danach wurde auf 75°C erhitzt und weitere 3 Stunden gerührt. Nach Kontrolle der vollständigen Umsetzung wurde das Reaktionsgemisch auf 30°C abgekühlt und 115g Wasser zugegeben.

Das Gemisch wurde bis zum vollständigen Lösen des Feststoffes (Ammoniumchlorid) gerührt. Danach wurde die organische Phase abgetrennt und die verbleibende wässrige Phase mit 18g Hexan extrahiert. Der organische Extrakt wurde mit der zuvor erhaltenen Produktphase vereinigt und zunächst bei Normaldruck zur Abtrennung von Ethanol, Wasser und Hexan destilliert. Anschließend wurde bei 10 mbar bis zum Erreichen einer konstanten Siedetemperatur von 128°C andestilliert.

Der verbleibende Sumpf enthielt 97,8% w/w 2,2-Dichlorphenylessigsäureethylester.

Ausbeute: 83,6g 2,2-Dichlorphenylessigsäureethylester (95% d.Th.) 97,8% w/w

### Beispiel 3: Herstellung von 2,2-Dichlorphenylessigsäureethylester aus 2,2-Dichlorphenylacetonitril (2. Stufe Labor (Variante II))

123,57g einer aus einer Chlorierung analog Beispiel 1 erhaltenen ethanolischen Salzsäure (30,22g = 0,83 mol = 2,18Äq. Salzsäure; 6,77g = 3,76 mol = 0,99 Äq. Wasser; 86,6g = 1,88 mol = 4,95 Äq. Ethanol) wurden auf 40°C erwärmt.

Dann wurden innerhalb von 30 Minuten 70,0g (0,38 mol) 2,2-Dichlorphenylacetonitril bei 40°C zudosiert und nach erfolgter Zugabe weitere 2 Stunden bei 40°C gerührt. Danach wurde auf 75°C erhitzt und weitere 3 Stunden gerührt. Nach Kontrolle der vollständigen Umsetzung wurde das Reaktionsgemisch auf 30°C abgekühlt und 115g Wasser zugegeben.

Das Gemisch wurde bis zum vollständigen Lösen des Feststoffes (Ammoniumchlorid) gerührt. Danach wurde die organische Phase abgetrennt und die verbleibende wässrige Phase mit 18g Hexan extrahiert. Der organische Extrakt wurde mit der zuvor erhaltenen Produktphase vereinigt und zunächst das Wasser am Wasserabscheider abgetrennt. Danach wurde bei Normaldruck zur Abtrennung von Ethanol und Hexan destilliert. Anschließend wurde bei 10 mbar bis zum Erreichen einer konstanten Siedetemperatur von 128°C andestilliert.

Der verbleibende Sumpf enthielt 93,9% w/w 2,2-Dichlorphenylessigsäureethylester Zur weiteren Reinigung wurde das Produkt über Kopf destilliert.

Ausbeute: 70g 2,2-Dichlorphenylessigsäureethylester (80% d.Th.) 99,4%w/w

### Beispiel 4: Pilotierungsversuch Chlorierung Benzylcyanid zu 2,2-Dichlorphenylacetonitril

400kg (3385 mol) Benzylcyanid wurden in einen zuvor inertisierten Email-Kessel chargiert, danach wurden während einer Stunde bei 40-50°C 40kg (1096 mol = 0,324 Äq.) Salzsäuregas bei geöffnetem Abgasventil eingeleitet und dann das Abgasventil geschlossen.

Anschließend wurden 487kg (6859 mol = 2,026 Äq.) Chlor über 15 Stunden bei 55-60°C und 3-3,5 bar eingeleitet.

Nach Ende des Chloreinleitens wurde das Abgasventil geschlossen und 3 Stunden bei 60-63°C gerührt. Danach wurde der Kessel entspannt, und zum Austreiben von Chlor und HCl-Gas Stickstoff durchgeleitet.

Der Abgaswäscher wurde mit 475kg (10326 mol = 3,05Äq.) Ethanol und 94kg konzentrierter Salzsäure (3394 mol = 1 Äq. = 61,1kg Wasser und 901 Mol = 0,266 Äq. = 32,9kg Salzsäure) beschickt und das Abgas aus der Chlorierung bei 10-15°C bis zu einer Chlorkonzentration von max. 3% im Abgas eingeleitet. Dahinter war ein Sicherheitswäscher (mit 10%iger Natronlauge beschickt) geschaltet.

Das restliche Abgas wurde direkt in den oben beschriebenen Natronlauge-Wäscher eingeleitet.

### Ausbeute:

636kg 2,2-Dichlorphenylacetonitril (∼theoretische Ausbeute)
825kg 35,2%ige ethanolische Salzsäure (Ausbeute an HCl aus der Chlorierung: 89% d.Th.)

### Beispiel 5: Herstellung von 2,2-Dichlorphenylessigsäureethylester aus 2,2-Dichlorphenylacetonitril (2. Stufe Pilotierung (Variante II))

410kg der gemäß Beispiel 4 erhaltenen ethanolischen Salzsäure (143,5kg = 3932 mol = 2,31Äq. Salzsäure; 30,5kg = 1694 mol = 1 Äq. Wasser; 236kg = 5130 mol =3,02 Äq. Ethanol) wurden mit 100kg (2174 mol = 1,28 Äq.) Ethanol verdünnt und auf 40°C erwärmt.

Dann wurden innerhalb von 2 Stunden 316kg (1699 mol) 2,2-Dichlorphenylacetonitril bei 40°C zudosiert und nach erfolgter Zugabe eine weitere Stunde bei 40°C gerührt. Danach wurde auf 70°C erhitzt und weitere 6 Stunden gerührt. Nach Kontrolle der vollständigen Umsetzung wurde das Reaktionsgemisch auf 30°C abgekühlt.

Die erhaltene Suspension wurde in 570I Wasser eingetragen und bis zum vollständigen Lösen des Feststoffes (Ammoniumchlorid) gerührt. Danach wurde die organische Phase abgetrennt und die verbleibende wässrige Phase mit 80kg Hexan extrahiert. Der organische Extrakt wurde mit der zuvor erhaltenen Produktphase vereinigt und zunächst bei Normaldruck bis 90°C andestilliert (zur Abtrennung von Ethanol, Wasser und Hexan) und anschließend bei 7 mbar bis 135°C fraktioniert.

Ausbeute: 325kg 2,2-Dichlorphenylessigsäureethylester (82% d.Th.) 98,8% w/w

### Beispiel 6: Herstellung von 2,2-Dichlorphenylessigsäureethylester aus 2,2-Dichlorphenylacetonitril (2. Stufe Pilotierung (Variante I))

325,3kg (1749 mol) 2,2-Dichlorphenylacetonitril wurden mit 100kg (2174 mol = 1,28 Äq.) Ethanol verdünnt und auf 40°C erwärmt.

Dann wurden innerhalb von 3 Stunden 410kg der gemäß Beispiel 4 erhaltenen ethanolischen Salzsäure (143,5kg = 3932 mol = 2,25Äq. Salzsäure; 30,5kg = 1694 mol = 0,97 Äq. Wasser; 236kg = 5130 mol = 2,93 Äq. Ethanol) bei 40°C zudosiert und nach erfolgter Zugabe weitere 3 Stunden bei 40°C gerührt. Danach wurde auf 70°C erhitzt und weitere 6 Stunden gerührt. Nach Kontrolle der vollständigen Umsetzung wurde das Reaktionsgemisch auf 30°C abgekühlt.

Die erhaltene Suspension wurde in 570I Wasser eingetragen und bis zum vollständigen Lösen des Feststoffes (Ammoniumchlorid) gerührt. Anschließend wurde die organische Phase abgetrennt und die verbleibende wässrige Phase mit 80kg Hexan extrahiert. Der organische Extrakt wurde mit der zuvor erhaltenen Produktphase vereinigt und zunächst bei 20-25°C und leichtem Unterdruck andestilliert (zur Abtrennung von Ethanol, Wasser und Hexan) und anschließend bei 7 mbar bis 135°C fraktioniert.

Ausbeute: 346,4kg 2,2-Dichlorphenylessigsäureethylester (85% d.Th.) 98,9% w/w

### Beispiel 7: Herstellung von 2,2-Dichlorphenylessigsäureethylester aus 2,2-Dichlorphenylacetonitril (2. Stufe Pilotierung (Variante II mit Rückveresterung))

375kg der bei der Chlorierung gemäß Beispiel 4 erhaltenen ethanolischen Salzsäure (136,7kg = 3745 mol = 2,16 Äq. Salzsäure; 28,2kg = 1567,5 mol = 0,91 Äq. Wasser; 210,1kg = 4567 mol =2,64 Äq. Ethanol) wurden mit 100kg (2174 mol = 1,26 Äq.) Ethanol verdünnt und auf 40°C erwärmt.

Dann wurden innerhalb von 2 Stunden 322kg (1732 mol) 2,2-Dichlorphenylacetonitril bei 40°C zudosiert und nach erfolgter Zugabe eine weitere Stunde bei 40°C gerührt. Danach wurde auf 70°C erhitzt und weitere 6 Stunden gerührt. Nach Kontrolle der vollständigen Umsetzung wurde das Reaktionsgemisch auf 30°C abgekühlt.

Die erhaltene Suspension wurde in 570I Wasser eingetragen und bis zum vollständigen Lösen des Feststoffes (Ammoniumchlorid) gerührt. Anschließend wurde die organische Phase abgetrennt und die verbleibende wässrige Phase mit 80kg Hexan extrahiert. Der organische Extrakt wurde mit der zuvor erhaltenen Produktphase vereinigt und zunächst bei Normaldruck bis 120°C andestilliert (zur Abtrennung von Ethanol, Wasser und Hexan). Da das Rohprodukt zu viel organische Säuren (aus Hydrolyse des Produktes) enthielt, wurden 40kg Hexan und 20kg Ethanol zugegeben und erneut bei Normaldruck bis 120°C destilliert. Anschließend wurde das Rohprodukt bei 7 mbar bis 135°C fraktioniert.

Ausbeute: 329kg 2,2-Dichlorphenylessigsäureethylester (81,5% d.Th.) 98,0% w/w

### Beispiel 8 Rückveresterung Labor

11,3g 2,2-Dichlorphenylessigsäureethylester mit einem Gehalt von 0,13% w/w Phenylglyoxylsäure (einem Hydrolyseprodukt von 2,2-Dichlorphenylessigsäureethylester) wurden mit 2ml Hexan und 0,5ml Ethanol für 2 Stunden auf 70°C erhitzt. Danach wurden die Lösungsmittel abdestilliert.

Ergebnis: Der Gehalt an Phenylglyoxylsäure sinkt auf 0,01% w/w

## Patentansprüche

1. **Verbessertes Verfahren zur Herstellung von 2,2-Dichloro- oder Dibromo-phenylessigsäurealkylester der Formel** 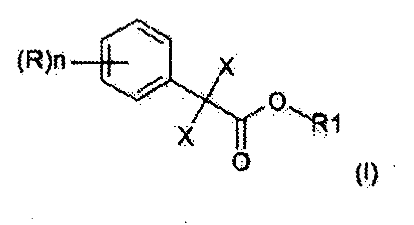 in der X Cl oder Br ist, n eine ganze Zahl von 1 bis 5 sein kann, R Wasserstoff, C₁-C₆-Alkyl, Aryl, Heteroaryl, C₁-C₆-Alkoxy, Aryloxy oder Halogen und R1 C₁-C₈-Alkyl bedeuten, **dadurch gekennzeichnet, dass** ein 2,2-Dichlor- oder Dibromphenylacetonitril der Formel 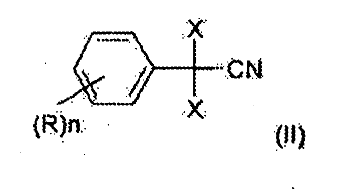 in der X, n und R wie oben definiert sind,
in 0,8 bis 2 mol Wasser pro mol Nitril der Formel (ii), 1 bis 8 mol Alkohol der Formel
R1OH (III)
in der R1 wie oben definiert ist, pro mol Nitril der Formel (II) und
in Gegenwart von 1 bis 3 mol HCl oder HBr pro mol Nitril der Formel (II), gegebenenfalls in Anwesenheit eines unter den Reaktionsbedingungen inerten Lösungsmittels zu dem entsprechenden 2,2-Dichloro- oder Dibromophenylessigsäurealkylester der Formel (I) umgesetzt wird, wobei die Reaktionstemperatur in der ersten Phase der Umsetzung bei 35 bis 55°C und in der zweiten Phase bei 60 bis 100°C liegt, worauf nach erfolgter Umsetzung das Reakitonsgemisch auf 20 bis 40°C abgekühit und mit Wasser verdünnt wird, und der entsprechende 2,2-Dichloro- oder Dibromo-phenylessigsäurealkylester der Formel (I) isoliert wird.

2. Verfahren nach Anspruch 1, **dadurch gekennzeichnet, dass** in der Formel (I) R Wasserstoff, C₁-C₄-Alkyl, C₁-C₄-Alkoxy, Phenyl, Phenoxy, Fluor, Brom oder Jod bedeutet und n, falls R nicht Wasserstoff ist, eine ganze Zahl von 1 bis 3 ist.

3. Verfahren nach Anspruch 1, **dadurch gekennzeichnet, dass** als Alkohol der Formel (III) Methanol, Ethanol oder n-Butanol eingesetzt wird.

4. Verfahren nach Anspruch 1, **dadurch gekennzeichnet, dass** für den Fall, dass der Alkohol der Formel (III) in einer Menge von 1 bis zu 3 mol pro mol Nitril der Formel (II) eingesetzt wird, die Umsetzung in Gegenwart eines unter den Reaktionsbedingungen inerten Lösungsmittel aus der Gruppe Methyl-tert.butylether, Diethylether, Tetrahydrofuran, Dioxan, Ethylenglykoldimethylether. Toluol, Hexan; Heptan, Dichlormethan oder Chlorbenzol durchgeführt wird.

5. Verfahren nach Anspruch 1, **dadurch gekennzeichnet, dass** als HCl oder HBr das HCl- bzw. HBr-Abgas, das bei der Umsetzung von gegebenenfalls substituierten Benzylcyanid der Formel 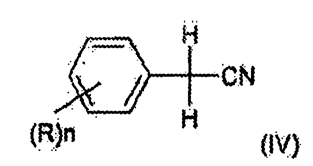 in der n und R wie in der Formel (I) definiert sind, mit Chlor, in Gegenwart katalytischer Mengen an Salzsäuregas, bzw. mit einem Bromierungsmittel zu dem korrespondierenden Nitril der Formel (II) erhalten wird, eingesetzt wird, wodurch eine direkte Kopplung der Herstellung des Nitrite der Formel (II) an die Herstellung des entsprechenden 2,2-Dichloro-oder Dibromo-phenylessigsäurealkylesters der Formel (I) erzielt wird.

6. Verfahren zur Herstellung von 2,2-Dichloro- oder Dibromo-phenylessigsäurealkylester der Formel (I), **dadurch gekennzeichnet, dass** in einer 1. Stufe ein gegebenenfalls substituiertes Benzylcyanid der Formel 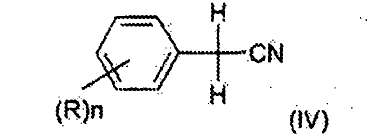 in der n eine ganze Zahl von 1 bis 5 sein kann und R Wasserstoff, C₁-C₈-Alkyl, Aryl, Heteroaryl, C₁-C₈-Alkoxy, Aryloxy oder Halogen und R1 C₁-C₈-Alkyl bedeutest,
mit Chlor, in Gegenwart katalytischer Mengen an Salzsäuregas, bzw, mit einem Bromierungsmittel zu dem korrespondierenden Nitril der Formel 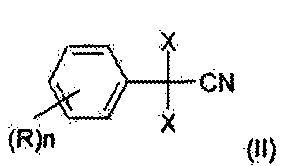 in der n und R wie oben definiert sind, und X Cl oder Br bedeutet,
umgesetzt wird und das sich bildende HCl- bzw, HBr-Abgas in der zweiten Stufe zur Umsetzung des Nitrils der Formen (II) zu dem entsprochenden 2,2-Dichloro- oder Dibromo-phenylessig-saürealkylester der Formel 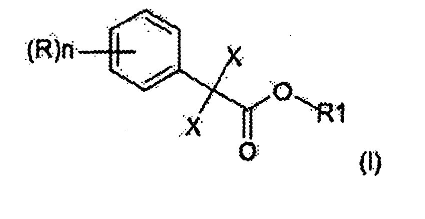 in der X, n und R wie oben definiert sind und R1 C₁-C₈-Alkyl bedeutet, eingesetzt wird, wobei die Umsetzung zu dem entsprechenden 2,2-Dichloro- oder Dibromo-phenylessigsäurealkylester der Formel (I) in einer zweiten Stufe in 0,8 bis 2 mol Wasser pro mol Nitril der Formel (II), 1 bis 8 mol Alkohol der Formel
R1OH (III)
in der R1 wie oben definiert ist, pro mol Nitril der Formel (II) und:
mit bis 3 mol HCl- bzw. HBr in Form des Abgases aus der 1. Stufe pro mol Nitril der Formel (II), gegebenenfalls in Anwesenheit eines unter den Reaktionsbedingungen inerten Lösungsmittels erfolgt und die Reaktionstemperatur in der ersten Phase der Umsetzung bei 35 bis 55°C und in der zweiten Phase bei 60 bis 100°C liegt, worauf nach erfolgter Umsetzung das Reaktionsgemisch auf 20 bis 40°C abgekühlt und mit Wasser verdünnt wird, und der entsprechende 2,2-Dichloro- oder Dibromo-phenylessigsäurealkylester der Formel (I) isoliert wird.

7. Verfahren nach Anspruch 1, **dadurch gekennzeichnet, dass** zur Umsetzung des Nitrils der Formel (II) ein Alkohol/Wasser/HCl- bzw. HBr-Gemisch verwendet wird, das durch Einleiten von HCl-Gas bzw. HBr-Gas in eine Mischung aus Wasser und Alkohol, oder durch Einleiten von HCl-Gas bzw. HBr-Gas in eine Lösung aus Alkohol und wässriger HCl bzw. HBr, oder durch Einleiten von HCl-Gas bzw. HBr-Gas in Alkohol mit anschließendem Verdünnen mit Wasser erhalten wird, wobei das gewünschte Molyerhältnis im Alkohol/Wasser/HCl- bzw. HBr-Gemisch gegebenenfalls durch Verdünnen der vorhandenen wässrigen, alkoholischen HCl- bzw. HBr-Lösung mit Alkohol und/oder Wasser eingestellt werden kann.

8. Verfahren nach Anspruch 7, **dadurch gekennzeichnet dass** als HCl-Gas bzw. HBr-Gas ein gemäß Anspruch 5 erhaltenes Abgas aus der Umsetzung eines Benzylcyanids der Formel (IV) zu dem korrespondierenden Nitril der Formel (II) eingesetzt wird, wodurch die Herstellung des Nitril der Formel (II) nicht direkt mit der Herstellung des 2,2-Dichloro- oder Dibromo-phenylessigsäurealkylesters der Formel (I) gekoppelt werden muss, und HCl bzw. HBr, die als Abgas bei der Herstellung des Nitrils der Formel (II) erhalten werden auch in Form des Alkohol/Wasser/HCl- bzw. HBr-Gemisches zwischengelagert werden können.

9. Verfahren nach Anspruch 1, **dadurch gekennzeichnet, dass** zur Isolierung der entsprechenden 2,2-Dichloro- oder Dibromo-phenylessigsäurealkylester der Formel (I) soviel Wasser zugesetzt wird, dass das ausgefallene Ammoniumchlorid bzw. -bromid gerade gelöst wird und eine Phasentrennung eintritt, worauf, gegebenenfalls nach Extraktion der wässrigen Phase, von der organischen Phase zunächst bei Normaldruck und einher Temperatur bis maximal 90°C Wasser, Alkohol und gegebenenfalls vorhandenes Extraktionsmittel und anschließend unter Vakuum Nebenprodukte bis zum Erreichen einer konstanten Siedetemperatur abdestilliert werden, sodass der entsprechende 2,2-Dichloro- oder Dibromophenylessigsäurealkylester der Formel (I) im Sumpf verbleibt, der gegebenenfalls zur weiteren Reinigung über Kopf destilliert werden kann, oder worauf, gegebenenfalls nach Extraktion der wassugen Phase, von der organischen Phase zuerst das Wasser am Wasserscheider abgetrennt wird und anschließend bei Normal-druck Alkohol und gegebenenfalls vorhandenes Extraktionsmittel abdestilliert wird.

10. Verfahren nach Anspruch 9, **dadurch gekennzeichnet, dass** der rohe 2,2-Dichloro- oder Dibromo-phenylessäurealkylester der Formel (I) für den Fall, dass dieser zuviel organische Säure enthält, mit einem Extraktionsmittel aus der Gruppe Hexan, Heptan, Toluol, Ether oder Ester und dem entsprechenden Alkohol der Formel (III) versetzt wird und erneut destillativ aufgearbeitet wird.

## Claims

1. Improved process for preparing alkyl 2,2-dichloro- or dibromophenylacetates of the formula 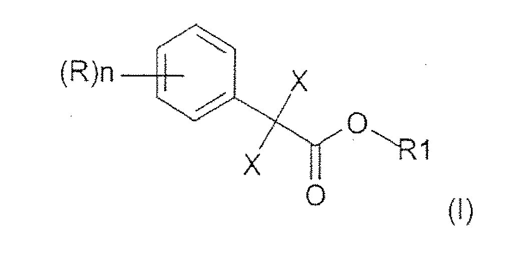 in which X is Cl or Br, n may be an integer from 1 to 5, R is hydrogen, C₁-C₈-alkyl, aryl, heteroaryl, C₁-C₈-alkoxy, aryloxy or halogen, and R1 is C₁-C₈-alkyl, **characterized in that** a 2,2-dichloro- or dibromophenylacetonitrile of the formula 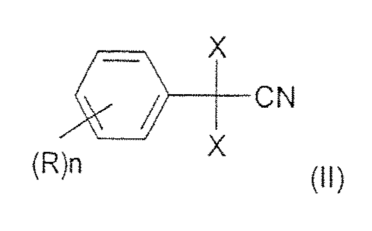 in which X, n and R are each as defined above,
in from 0.8 to 2 mol of water per mole of nitrile of the formula (II), from 1 to 8 mol of alcohol of the formula
R1OH (III)
in which R1 is as defined above, per mole of nitrile of the formula (II) and in the presence of from 1 to 3 mol of HCl or HBr per mole of nitrile of the formula (II), optionally in the presence of a solvent inert under the reaction conditions, is converted to the corresponding alkyl 2,2-dichloro- or dibromophenylacetate of the formula (I), the reaction temperature in the first phase of the conversion being from 35 to 55°C and, in the second phase, from 60 to 100°C, whereupon, on completion of conversion, the reaction mixture is cooled to from 20 to 40°C and diluted with water, and the corresponding alkyl 2,2-dichloro- or dibromophenylacetate of the formula (I) is isolated.

2. Process according to Claim 1, **characterized in that**, in the formula (I), R is hydrogen, C₁-C₄-alkyl, C₁-C₄-alkoxy, phenyl, phenoxy, fluorine, bromine or iodine, and n, if R is not hydrogen, is an integer from 1 to 3.

3. Process according to Claim 1, **characterized in that** the alcohol of the formula (III) used is methanol, ethanol or n-butanol.

4. Process according to Claim 1, **characterized in that**, in the case that the alcohol of the formula (II) is used in an amount of from 1 up to 3 mol per mole of nitrile of the formula (II), the reaction is carried out in the presence of a solvent, inert under the reaction conditions, from the group of methyl tert-butyl ether, diethyl ether, tetrahydrofuran, dioxane, ethylene glycol dimethyl ether, toluene, hexane, heptane, dichloromethane or chlorobenzene.

5. Process according to Claim 1, **characterized in that** the HCl or HBr used is the HCl or HBr offgas which is obtained in the reaction of optionally substituted benzene cyanide of the formula 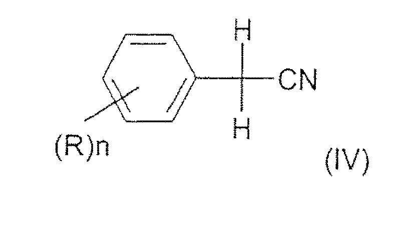 in which n and R are each as defined in the formula (I) with chlorine in the presence of catalytic amounts, or with a brominating agent, to the corresponding nitrile of the formula (II), which achieves direct coupling of the preparation of the nitrile of the formula (II) to the preparation of the corresponding alkyl 2,2-dichloro- or dibromophenylacetate of the formula (I).

6. Process for preparing alkyl 2,2-dichloro- or dibromophenylacetates of the formula (I), **characterized in that**, in a 1 st stage, an optionally substituted benzyl cyanide of the formula 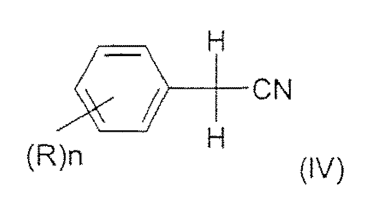 in which n may be an integer from 1 to 5 and R is hydrogen, C₁-C₈-alkyl, aryl, heteroaryl, C₁-C₈-alkoxy, aryloxy or halogen, and R1 is C₁-C₈-alkyl, is reacted with chlorine in the presence of catalytic amounts of hydrogen chloride gas, or with a brominating agent, to give the corresponding nitrile of the formula 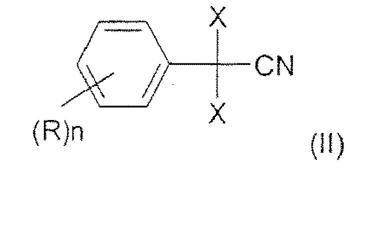 in which n and R are each as defined above and X is Cl or Br,
and the HCl or HBr offgas which forms is used in the second stage to convert the nitrile of the formula (II) to the corresponding alkyl 2,2-dichloro- or dibromophenylacetate of the formula 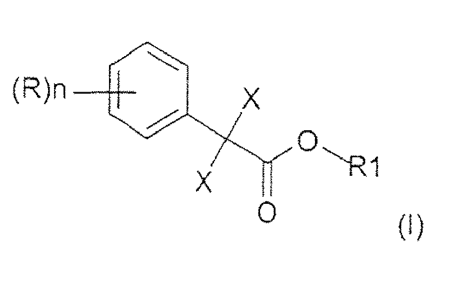 in which X, n and R are each as defined above and R1 is C₁-C₈-alkyl,
the conversion to the corresponding alkyl 2,2-dichloro- or dibromophenylacetate of the formula (I) in a second stage being effected in from 0.8 to 2 mol of water per mole of nitrile of the formula (II), from 1 to 8 mol of alcohol of the formula
R1OH (III)
in which R1 is as defined above, per mole of nitrile of the formula (II) and with from 1 to 3 mol of HCl or HBr in the form of the offgas from the 1st stage per mole of nitrile of the formula (II), optionally in the presence of a solvent inert under the reaction conditions, and the reaction temperature in the first phase of the conversion being from 35 to 55°C and, in the second phase, from 60 to 100°C, whereupon, on completion of conversion, the reaction mixture is cooled to from 20 to 40°C and diluted with water, and the corresponding alkyl 2,2-dichloro- or dibromophenylacetate of the formula (I) is isolated.

7. Process according to Claim 1, **characterized in that** the nitrile of the formula (II) is converted using an alcohol/water/HCl or HBr mixture which is obtained by passing HCl gas or HBr gas into a mixture of water and alcohol, or by passing HCl or HBr gas into a solution of alcohol and aqueous HCl or HBr, or by passing HCl or HBr gas into alcohol with subsequent dilution with water, and the desired molar ratio in the alcohol/water/HCl or HBr mixture can optionally be adjusted by diluting the aqueous, alcoholic HCl or HBr solution present with alcohol and/or water.

8. Process according to Claim 7, **characterized in that** the HCl gas or HBr gas used is an offgas, obtained according to Claim 5, from the conversion of a benzyl cyanide of the formula (IV) to the corresponding nitrile of the formula (II), as a result of which the preparation of the nitrile of the formula (II) does not have to be coupled directly with the preparation of the alkyl 2,2-dichloro- or dibromophenylacetate of the formula (I), and HCl or HBr which are obtained as offgas in the preparation of the nitrile of the formula (II) may also be intermediately stored in the form of the alcohol/water/HCl or HBr mixture.

9. Process according to Claim 1, **characterized in that** the corresponding alkyl 2,2-dichloro- or dibromophenylacetates of the formula (I) are isolated by adding sufficient water that the precipitated ammonium chloride or bromide is just dissolved and a phase separation occurs, whereupon, optionally after extraction of the aqueous phase, water, alcohol and any extractant present are initially distilled out of the organic phase at atmospheric pressure and a maximum temperature of 90°C, and subsequently by-products under reduced pressure until a constant boiling temperature is attained, so that the corresponding alkyl 2,2-dichloro- or dibromophenylacetate of the formula (I) remains in the residue which may optionally be distilled overhead for further purification, or whereupon, optionally after extraction of the aqueous phase, the water is first removed from the organic phase on a water separator and alcohol and any extractant present are subsequently distilled off at atmospheric pressure.

10. Process according to Claim 9, **characterized in that** the crude alkyl 2,2-dichloro- or dibromophenylacetate of the formula (I), in the case that it contains too much organic acid, is admixed with an extractant from the group of hexane, heptane, toluene, ethers or esters and the corresponding alcohol of the formula (III) and again worked up distillatively.

## Revendications

1. Procédé amélioré pour la préparation d'esters alkyliques de l'acide 2,2-dichlorophénylacétique ou de l'acide 2,2-dibromophénylacétique de formule (I) 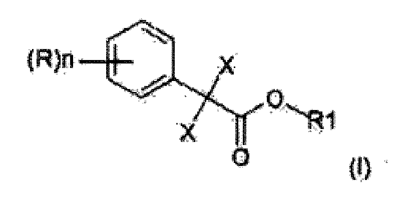 dans laquelle X représente Cl ou Br, n peut valoir un nombre entier de 1 à 5, R signifie hydrogène, C₁-C₁₈-alkyle, aryle, hétéroaryle, C₁-C₈-alcoxy, aryloxy ou halogène et R1 signifie C₁-C₈-alkyle, **caractérisé en ce qu'**on transforme un 2,2-dichlorophénylacétonitrile ou un 2,2-dibromophénylacétonitrile de formule 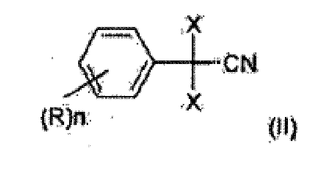 dans laquelle X, n et R sont définis comme ci-dessus, dans 0,8 à 2 moles d'eau par mole de nitrile de formule (II), 1 à 8 moles d'alcool de formule
R1OH (III)
dans laquelle R1 est défini comme ci-dessus, par mole de nitrile de formule (II) et
en présence de 1 à 3 moles de HCl ou de HBr par mole de nitrile de formule (II), le cas échéant en présence d'un solvant inerte dans les conditions de réaction, en l'ester alkylique de l'acide 2,2-dichlorophénylacétique ou de l'acide 2,2-dibromophénylacétique de formule (I) correspondant, la température de réaction dans la première phase de la transformation étant de 35 à 55°C et, dans la deuxième phase, de 60 à 100°C, suite à quoi, après la transformation, le mélange réactionnel est refroidi à 20 jusqu'à 40°C et dilué à l'eau et l'ester alkylique de l'acide 2,2-dichlorophénylacétique ou de l'acide 2,2-dibromophénylacétique de formule (I) correspondant est isolé.

2. Procédé selon la revendication 1, **caractérisé en ce que**, dans la formule (I), R signifie hydrogène, C₁-C₄-alkyle, C₁-C₄-alcoxy, phényle, phénoxy, fluor, brome ou iode et n, si R ne représente pas hydrogène, vaut un nombre entier de 1 à 3.

3. Procédé selon la revendication 1, **caractérisé en ce qu'**on utilise, comme alcool de formule (III), du méthanol, de l'éthanol ou du n-butanol.

4. Procédé selon la revendication 1, **caractérisé en ce que**, dans le cas où l'alcool de formule (III) est utilisé en une quantité de 1 à 3 moles par mole de nitrile de formule (II), la transformation est réalisée en présence d'un solvant inerte dans les conditions de réaction du groupe formé par le méthyl-tert-butyléther, le diéthyléther, le tétrahydrofuranne, le dioxane, l'éthylèneglycoldiméthyléther, le toluène, l'hexane, l'heptane, le dichlorométhane ou le chlorobenzène.

5. Procédé selon la revendication 1, **caractérisé en ce qu'**on utilise, comme HCl ou HBr, l'effluent gazeux de HCl ou, selon le cas, de HBr, qui est obtenu lors de la transformation de cyanure de benzyle le cas échéant substitué de formule 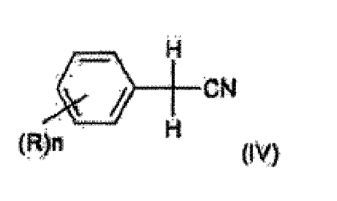 dans laquelle n et R sont comme définis dans la formule (I), avec du chlore en présence de quantités catalytiques d'acide chlorhydrique gazeux ou, selon le cas, avec un agent de bromuration, en nitrile correspondant de formule (II), ce qui permet d'obtenir un couplage direct de la préparation du nitrile de formule (II) à la préparation de l'ester alkylique de l'acide 2,2-dichlorophénylacétique ou de l'acide 2,2-dibromophénylacétique de formule (I) correspondant.

6. Procédé pour la préparation d'esters alkyliques de l'acide 2,2-dichlorophénylacétique ou de l'acide 2,2-dibromophénylacétique de formule (I), **caractérisé en ce que**, dans une 1ère étape, on transforme un cyanure de benzyle le cas échéant substitué de formule 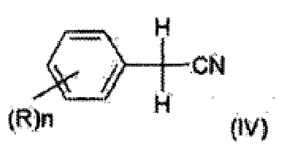 dans laquelle n peut valoir un nombre entier de 1 à 5 et R signifie hydrogène, C-C₈-alkyle, aryle, hétéroaryle, C₁-C₈-alcoxy, aryloxy ou halogène et R1 signifie C₁-C₈-alkyle,
avec du chlore en présence de quantités catalytiques d'acide chlorhydrique gazeux ou, selon le cas, avec un agent de bromuration, en nitrile correspondant de formule 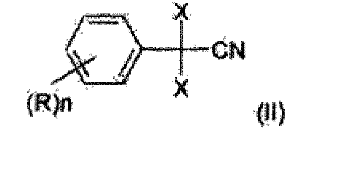 dans laquelle n et R sont définis comme ci-dessus et X signifie Cl ou Br,
et l'effluent gazeux de HCl ou, selon le cas, de HBr qui se forme est utilisé dans la deuxième étape pour la transformation du nitrile de formule (II) en ester alkylique de l'acide 2,2-dichlorophénylacétique ou de l'acide 2,2-dibromophénylacétique correspondant de formule 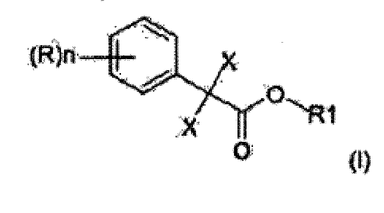 dans laquelle X, n et R sont définis comme ci-dessus et R1 signifie C₁-C₈-alkyle,
la transformation en ester alkylique de l'acide 2,2-dichlorophénylacétique ou de l'acide 2,2-dibromophénylacétique de formule (I) correspondant ayant lieu dans une deuxième étape dans 0,8 à 2 moles d'eau par mole de nitrile de formule (II), 1 à 8 moles d'alcool de formule
R1OH (III)
dans laquelle R1 est défini comme ci-dessus, par mole de nitrile de formule (II) et
avec 1 à 3 moles de HCl ou, selon le cas, de HBr sous forme de l'effluent gazeux de la 1ère étape par mole de nitrile de formule (II), le cas échéant en présence d'un solvant inerte dans les conditions de réaction, et la température de réaction dans la première phase de la transformation est de 35 à 55°C et, dans la deuxième phase, de 60 à 100°C, suite à quoi, après la transformation, le mélange réactionnel est refroidi à 20 jusqu'à 40°C et dilué à l'eau et l'ester alkylique de l'acide 2,2-dichlorophénylacétique ou de l'acide 2,2-dibromophénylacétique de formule (I) correspondant est isolé.

7. Procédé selon la revendication 1, **caractérisé en ce qu'**on utilise, pour la transformation du nitrile de formule (II), un mélange d'alcool/eau/HCl ou, selon le cas, HBr, qui est obtenu par introduction de HCl ou, selon le cas, de HBr gazeux dans un mélange d'eau et d'alcool ou par introduction de HCl ou, selon le cas, de HBr gazeux dans une solution d'alcool et de HCl ou, selon le cas, de HBr aqueux ou par introduction de HCl ou, selon le cas, de HBr gazeux dans de l'alcool avec une dilution consécutive à l'eau, le rapport molaire souhaité dans le mélange d'alcool/eau/HCl ou, selon le cas, HBr pouvant être réglé le cas échéant par dilution de la solution aqueuse, alcoolique de HCl ou, selon le cas, de HBr qui existe avec de l'alcool et/ou de l'eau.

8. Procédé selon la revendication 7, **caractérisé en ce qu'**on utilise, comme HCl gazeux ou, selon le cas, HBr gazeux, un effluent gazeux obtenu selon la revendication 5 provenant de la transformation d'un cyanure de benzyle de formule (IV) en nitrile correspondant de formule (II), la préparation du nitrile de formule (II) ne devant pas être couplée directement à la préparation de l'ester alkylique de l'acide 2,2-dichlorophénylacétique ou de l'acide 2,2-dibromophénylacétique de formule (I), et le HCl ou, selon le cas, le HBr, qui sont obtenus sous forme d'effluent gazeux lors de la préparation du nitrile de formule (II), peuvent également être entreposés de manière intermédiaire sous forme du mélange d'alcool/eau/HCl ou, selon le cas, HBr.

9. Procédé selon la revendication 1, **caractérisé en ce que**, pour l'isolement de l'ester alkylique de l'acide 2,2-dichlorophénylacétique ou de l'acide 2,2-dibromophénylacétique de formule (I) correspondant, on ajoute une quantité d'eau telle que le chlorure ou, selon le cas, le bromure d'ammonium précipité est précisément dissous et qu'une séparation de phases se produit, suite à quoi, le cas échéant après extraction de la phase aqueuse, on élimine par distillation de la phase organique, d'abord à pression normale et à une température jusqu'au maximum 90°C, l'eau, l'alcool et l'agent d'extraction le cas échéant présent, puis sous vide, les produits secondaires jusqu'à atteindre une température d'ébullition constante, de manière telle que l'ester alkylique de l'acide 2,2-dichlorophénylacétique ou de l'acide 2,2-dibromophénylacétique de formule (I) correspondant reste dans le produit de fond qui peut le cas échéant être distillé via la tête pour une purification supplémentaire ou, suite à quoi, le cas échéant après extraction de la phase aqueuse, on sépare d'abord de la phase organique l'eau sur un séparateur d'eau puis on élimine par distillation à pression normale l'alcool et l'agent d'extraction le cas échéant présent.

10. Procédé selon la revendication 9, **caractérisé en ce que** l'ester alkylique de l'acide 2,2-dichlorophénylacétique ou de l'acide 2,2-dibromophénylacétique de formule (I) brut, dans le cas où celui-ci contient trop d'acide organique, est additionné d'un agent d'extraction du groupe formé par l'hexane, l'heptane, le toluène, les éthers ou les esters et de l'alcool correspondant de formule (III) et à nouveau traité par distillation.
